# EUROPEAN PATENT APPLICATION

(11) **EP 2 392 341 A1**
(43) Date of publication of application: **07.12.2011**
(21) Application number: 11168380.1
(22) Date of filing: 01.06.2011
(51) Int. Cl.: A61K 38/06, A61K 9/00, A61K 9/20, A61P 35/00

(54) **Compositions for use in treating myelodysplastic syndrome**

(30) Priority: 07.06.2010 US 352371 P; 16.05.2011 US 108752
(71) Applicant: Telik, Inc., Palo Alto, CA 94304 (US)
(72) Inventor: Brown, Gail L., Palo Alto, CA California 94304 (US)
(74) Representative: Gibson, Mark

(57) **Abstract**

The invention relates to compositions for use in specific treatment regimens for treating myelodysplastic syndrome.

## Description

### Field of the Invention

The invention relates to compositions for use in treating myelodysplastic syndrome.

### Background of the Invention

Myelodysplastic syndrome(s) (MDS) refers to a heterogeneous group of clonal hematopoietic stem cell disorders characterized by ineffective hematopoiesis (blood cell production) involving one or more cell lineages (red blood cells, white blood cells or platelets) and a variable risk of transformation to acute myeloid leukemia (AML). This syndrome becomes more common with age. It is estimated that MDS affects approximately 300,000 people worldwide. According to the American Cancer Society, 10,000 to 20,000 new cases of MDS are diagnosed each year in the United States alone. Survival rates using current therapy range from 6 months to 6 years with patients often requiring blood transfusions to manage their disease.

Currently, there are three approved drugs for treating MDS by the U.S. Food and Drug Administration (FDA). Lenalidomide is indicated for the treatment of transfusion dependent MDS patients with del(5q) and lower risk disease while azacytidine and decitabine are approved for all categories. With the exception of del(5q) patients, the response rate is approximately 50%, highlighting the need for clinical trials of new agents.

Ezatiostat and its salts are disclosed in US Patent No. 5,763,570. Ezatiostat has the IUPAC chemical name of ethyl (2S)-2-amino-5-[[(2*R*)-3-benzylsulfanyl-1-[[(1*R*)-2-ethoxy-2-oxo-1-phenylethyl]amino]-1-oxopropan-2-yl]amino]-5-oxopentanoate.

One example of a salt of ezatiostat is the hydrochloride salt, ezatiostat hydrochloride (USAN), which has the molecular weight of 566.1, the trademark of Telintra®, and the CAS registry number of 286942-97-0. U.S. Patent Application No. 13/041,136, filed March 4, 2011, describes ansolvate and polymorphs of ezatiostat hydrochloride.

Ezatiostat hydrochloride has been evaluated for the treatment of MDS, in a Phase I-Ila study using a liposomal formulation (US Patent No. 7,029,695), as reported at the 2005 Annual Meeting of the American Society for Hematology (Abstract #2250) and by Raza et al. in Journal of Hematology & Oncology, 2:20 (published online on 13 May 2009); and in a Phase I study using a tablet formulation, as reported at the 2007 Annual Meeting of the American Society for Hematology (Abstract #1454) and by Raza et al. in Blood, 113:6533-6540 (prepublished online on 27 April 2009), and in a single patient case report by Quddus et a/. in Journal of Hematology & Oncology, 3:16 (published online on 23 April 2010).

### Summary of the Invention

In one embodiment, the invention is directed to a composition comprising a therapeutically effective amount of ezatiostat or a salt thereof for use in treating a myelodysplastic syndrome in a patient wherein said composition is orally administered to said patient in an amount of ezatiostat or a salt thereof equivalent to up to about 2 grams per day of ezatiostat hydrochloride for at least 2 weeks. In a preferred embodiment, the dosing of ezatiostat or a salt thereof is a therapeutically effective amount of up to about 1 gram ezatiostat hydrochloride (or equivalent) administered twice a day (b.i.d.). In one embodiment, ezatiostat or a salt thereof is administered orally.

In an embodiment of the invention, the patient is treated with ezatiostat or a salt thereof equivalent to up to about 2 grams per day of ezatiostat hydrochloride for three weeks followed by a week interruption without ezatiostat treatment. After the fourth week, the regimen can be repeated as necessary. Preferably, the ezatiostat or a salt thereof is administered twice a day, for example in equal doses. Thus, according to one aspect of the invention there is provided a composition comprising ezatiostat or a salt thereof for use in treating myelodysplastic syndrome in a patient wherein said composition is administered in an amount of ezatiostat or a salt thereof equivalent to 1 gram dosages of ezatiostat hydrochloride twice a day for three weeks followed by a week where no ezatiostat or a salt thereof is administered.

In another embodiment of the invention, the patient is treated continuously with a therapeutically effective amount of ezatiostat or a salt thereof equivalent to up to about 2 grams per day of ezatiostat hydrochloride preferably administered in divided doses twice a day. It is contemplated that in this embodiment, the therapeutically effective amount of ezatiostat or a salt thereof may be less than that when there is a week interruption in the treatment regimen.

In one embodiment, ezatiostat or a salt thereof can be administered as a tablet formulation. Such a tablet formulation is disclosed in U.S. Patent Application No. 13/075,116, filed March 29, 2011, titled "TABLET FORMULATION OF EZATIOSTAT".

These and other embodiments of the invention are further described in the text that follows.

### Detailed Description of the Invention

As per above, the invention is directed to a treatment regimen for treating MDS using ezatiostat or a salt thereof. However, prior to describing the invention in detail, the following terms will be defined.

It must be noted that as used herein and in the appended claims, the singular forms "a", "an", and "the" include plural referents unless the context clearly dictates otherwise. Thus, for example, reference to "a pharmaceutically acceptable excipient" includes a plurality of pharmaceutically acceptable excipients.

The term "comprising" or "comprises" means that the compositions and uses include the recited elements, but do not exclude others. "Consisting essentially of" when used to define compositions and uses, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of' means excluding more than trace elements of other ingredients and substantial method steps. Embodiments defined by each of these transition terms are within the scope of the invention.

As used herein, the term "treatment" or "treating" means any treatment of MDS in a patient which produces one or more of the following:
● inhibiting the disease or condition, that is, arresting or suppressing the development of symptoms (e.g., need for blood transfusion, abnormal blood count, and the like); and/or
● relieving the disease or condition that is, causing the regression of symptoms.

As used herein, the term "patient" refers to mammals and includes humans and non-human mammals.

The term "about" when used before a numerical designation, e.g., temperature, time, amount, and concentration, including range, indicates approximations which may vary by (+) or (-)15%, 10%, 5% or 1 %.

The term "therapeutically effective amount" refers to the amount of ezatiostat or a salt thereof that is an amount sufficient to effect treatment, as defined herein, when administered to a subject in need of such treatment. In one embodiment, the therapeutically effective amount will be ezatiostat or a salt thereof equivalent to up to about 2 grams per day of ezatiostat hydrochloride administered per day. Preferably, ezatiostat or a salt thereof is administered in an amount of 2 grams per day and, more preferably, is administered twice a day in equal 1 gram doses. Such a therapeutically effective amount is particularly relevant when the treatment regimen is for 3 weeks of administration of ezatiostat or a salt thereof followed by a week of no administration of the drug.

It is contemplated, however, that therapeutically effective amounts used in continuous administration may be lower than the amounts used during a three week treatment cycle followed by a week of no administration.

While twice a day administration is preferred, it is contemplated that once a day administration or 3 times a day administration could be employed. In the former case, once a day administration would assist in patient compliance; whereas in the latter case, smaller tablets could be used for those patients who have difficulty with swallowing larger tablets.

It is to be understood that the invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

### Compositions of the Invention

In an embodiment, the invention is directed to a composition comprising a therapeutically effective amount of ezatiostat or a salt thereof for use in treating a myelodysplastic syndrome in a patient wherein said composition is orally administered to said patient in an amount of ezatiostat or a salt thereof equivalent to up to about 2 grams per day of ezatiostat hydrochloride for at least 2 weeks. In a preferred embodiment, the dosing of ezatiostat or a salt thereof is a therapeutically effective amount of up to about 1 gram ezatiostat hydrochloride (or equivalent) administered in two separate doses per day.

In one embodiment of the invention, ezatiostat or a salt thereof is administered in 1 gram dosages twice a day for three weeks followed by an interruption of 1 week. After the interruption, the regimen can be repeated as necessary. This regimen may be referred to as the "three-week regimen."

In another embodiment of the invention, the patient is treated continuously with a therapeutically effective amount of ezatiostat or a salt thereof equivalent to up to about 2 grams per day of ezatiostat hydrochloride, preferably in two divided doses, for at least two weeks. In n this embodiment, ezatiostat or a salt thereof can be administered so long as the patient is in need of and can tolerate such treatment. It is contemplated that in this embodiment, the therapeutically effective amount of ezatiostat or a salt thereof may be less than that when there is an interruption in the treatment regimen. This regimen may be referred to as the "continuous regimen."

The treatment with ezatiostat or a salt thereof may involve one or a combination of two or more of the dosing regimens described herein. The following are exemplifying dosing schedules of ezatiostat hydrochloride:
● 1 gram ezatiostat hydrochloride (or equivalent of ezatiostat or another salt) administered twice per day for 3 weeks for an aggregate total dosing of 42 grams followed by a week when no ezatiostat or a salt is administered;
● 1 gram ezatiostat hydrochloride (or equivalent of ezatiostat or another salt) administered twice per day continuously until the attending clinician deems it appropriate for the patient to be withdrawn from administration;
● a therapeutically effective amount of up to 2 grams of ezatiostat hydrochloride (or equivalent of ezatiostat or another salt) per day administered in one, two, or three divided doses for 3 weeks followed by a week when no ezatiostat or a salt is administered; and/or
● a therapeutically effective amount of up to 2 grams of ezatiostat hydrochloride (or equivalent of ezatiostat or another salt) per day administered in one, two, or three divided doses continuously until the attending clinician deems it appropriate for the patient to be withdrawn from administration.

Ezatiostat hydrochloride in the above dosings can be replaced with an equivalent amount (in terms of ezatiostat content) of ezatiostat itself or another salt of ezatiostat.

In the above treatment regimens, ezatiostat or a salt thereof can be delivered as a tablet. Examples of such a tablet formulation are disclosed in U.S. Patent Application No. 13/075,116, filed March 29, 2011, titled "TABLET FORMULATION OF EZATIOSTAT".

When administration of ezatiostat or a salt thereof is twice a day, it is preferred that the interval between the first and second doses be from about 6 to 14 hours and more preferably between abut 8 and 14 hours.

### Example

The present invention is further defined by reference to the following examples.

Eighty-seven patients were randomized and treated at 23 investigational sites. After initial dose ranging in 14 patients, two dose levels were selected for further study. Subsequently, 37 patients were treated at 3 grams daily for two weeks followed by a one week rest period, and 36 patients were treated at 2 grams daily for three weeks followed by a one week rest period. The data on these 73 patients was pooled for this preliminary analysis.

The median age was 72 years, with a patient population distribution of International Prognostic Scoring System (IPSS) low risk (23 patients, 32 %) and intermediate-1 risk (50 patients, 68 %). Patients had received a median of three prior MDS therapies including, 34 patients (47 %) with prior Revlimid® (lenalidomide) and 28 patients (38 %) with prior DNA methyltransferase inhibitors (DMTI) [azacitidine, decitabine].

At the time of preliminary analysis, 8 patients remained on treatment for continuing clinical benefit. The overall Hematologic Improvement - Erythroid (HI-E) rate was 22 %, 13 of 60 evaluable patients (95 % CI, 12.1-34.2). The median duration of HI-E response was 46 weeks (range 2-51). The median hemoglobin level increased by 2.0 gram/dL in responders. Eleven of 38 red blood cell (RBC) transfusion-dependent patients (29 %) had clinically significant RBC transfusion reductions (reduction of 4U/8 weeks, IWG 2006) with 4 patients (11 %) achieving RBC transfusion independence and 3 patients continuing on treatment. In addition, one patient continued in complete remission for more than 12 months following discontinuation of therapy (Quddus et al., J. Hem. and Onc. Apr. 2010, 3:15).

Telintra® continues to demonstrate multilineage hematologic improvement. There was a 15 % Hematologic Improvement - Neutrophil (HI-N) rate observed in 3 of 20 patients (95 % Cl, 3.2-37.9), and the bilineage HI rate (HI-E and HI-N) was 11%, 2 of 19 patients (95 % CI, 1.3-33.1).

There were three cytogenetic complete responses, one in a patient with 45X,-Y[4], 46, XY [16] abnormal cytogenetics that converted to normal after four cycles of therapy. Of the four patients enrolled in the study with del 5q minus, two had a complete cytogenetic response, including one who had failed prior Revlimid® therapy.

A planned logistic regression analysis was used to evaluate all known prognostic characteristics in order to define those patients who had an increased likelihood of HI-E response to Telintra®. Prior DMTI treatment predicts a five-fold decrease in the odds for a HI-E response to Telintra® (p=0.023). Prior Revlimid® treatment was observed to enhance HI-E response to Telintra®.
● There was a 40 % HI-E rate (6 of 15 patients, 95 % CI, 16.3 %-67.7 %) in patients who had prior Revlimid® treatment, but no prior DMTI treatment. Within this patient group, five of 11 patients (45 %) achieved significant RBC transfusion reduction with three of those patients (27 %) achieving transfusion independence.
● There was a 26% HI-E rate (6 of 23 patients, 95 % CI, 10.2 %-48.4 %) in patients who had no prior Revlimid® treatment and no prior DMTI treatment. Within this group, five of 11 patients (45%) achieved significant RBC transfusion reduction.
● There was a 0 % HI-E rate (0 of 17 patients, 95 % CI, 0 %-19.5 %) in patients who had no prior Revlimid® treatment but who had received prior DMTI treatment.

More than 403 cycles of Telintra® therapy have been administered. The safety data is based on all patients treated. The most common non-hematologic adverse events (AEs) were Grade 1 and 2 gastrointestinal (GI) respectively, nausea (45%, 16%), diarrhea (25%, 7%) and vomiting (30%, 12%). Grade 3 events were uncommon: nausea (1%), diarrhea (3%) and vomiting (2%). Prior DMTI treatment was associated with an increased incidence of GI AEs.

Telintra® treatment may result in clinically significant hematologic improvement in patients with MDS and may offer an alternative to RBC transfusions. These results are consistent with levels of efficacy observed in prior studies with Telintra®, the first GST P1-1 enzyme inhibitor tested in MDS patients.

The following table summarizes the results of this clinical study.

The data show both administration of 2 grams of ezatiostat hydrochloride per day over a 3 week period and administration of 3 grams of ezatiostat hydrochloride per day over a 2 week period provided efficacy in treating MDS. Furthermore and unexpectedly, the data show that administration of 2 grams of drug per day over a 3 week period gave a duration of response of 46.1 weeks versus the 18.4 weeks when 3 grams of ezatiostat hydrochloride per day were administered over a 2 week period.

**Table 1**

| Hematological Improvement—Erythroid (HI-E): Time to Response and Duration of Response Starting Telintra® Dose of 3,000 mg/day (1.5 g b.i.d.) or 2,000 mg/day (1 g b.i.d.) (Efficacy Evaluable Population) | | |
|---|---|---|
| | Telintra® Dosing Schedule | |
| | 1.5 g b.i.d. 2 weeks on & 1 week off (N=29) | 1 g b.i.d. 3 weeks on & 1 week off (N=31) |
| Time to HI-E Response (Weeks) [1] | | |
| N | 7 | 6 |
| Mean | 8.4 (0.72) | 8.9 (1.29) |
| Median | 8.1 | 8.4 |
| Min, Max | 8.0, 10.0 | 8.0, 11.3 |
| Duration of HI-E Response (Weeks) [2] | | |
| # Event | 5 (71.4 %) | 2 (33.3%) |
| # Censored | 2 (28.6 %) | 4 (66.7%) |
| Median (95 % CI) | 18.4 (3.1-51.0) | 46.1 (10.0-46.1) |
| Min, Max | 1.9-51.0 | 2.4-46.1 |

| | | |
|---|---|---|
| [1] Days from date of first dose of study medication to the date of first documentation of response plus one divided by 7. [2] Total number of days of where response is seen divided by 7. | | |

## Claims

1. A composition comprising a therapeutically effective amount of ezatiostat or a salt thereof for use in treating a myelodysplastic syndrome in a patient wherein said composition is orally administered to said patient in an amount of ezatiostat or a salt thereof equivalent to up to about 2 grams per day of ezatiostat hydrochloride for at least 2 weeks.

2. The composition of Claim 1 where the ezatiostat or a salt thereof is ezatiostat hydrochloride.

3. The composition of Claim 1, wherein said therapeutically effective amount of ezatiostat hydrochloride is 2 grams per day.

4. The composition of Claim 3, wherein the ezatiostat hydrochloride is administered in two separate 1 gram doses per day.

5. The composition of Claim 4, wherein the second dose is administered at an interval of from 6 to 14 hours after the first dose.

6. A composition comprising ezatiostat or a salt thereof for use in treating myelodysplastic syndrome in a patient wherein said composition is administered in an amount of ezatiostat or a salt thereof equivalent to 1 gram dosages of ezatiostat hydrochloride twice a day for three weeks followed by a week where no ezatiostat or a salt thereof is administered.

7. The composition of Claim 6, wherein after the fourth week, the treatment regimen with ezatiostat or a salt thereof is repeated.

8. The composition of any one of Claims 1-7 above, wherein the ezatiostat or a salt thereof is administered in a tablet form.
